# EUROPEAN PATENT APPLICATION

(11) **EP 0 829 461 A1**
(43) Date of publication of application: **18.03.1998**
(21) Application number: 97115568.4
(22) Date of filing: 08.09.1997
(51) Int. Cl.: C07C 17/12, C07C 25/08, C07C 25/10

(54) **Selective production of 1,4-dichloro-benzene and 1,2,4-trichlorobenzene**

(30) Priority: 09.09.1996 US 709959; 04.08.1997 US 905838
(71) Applicant: Occidental Chemical Corporation, Niagara Falls, N.Y. 14302 (US)
(72) Inventor: Krishnamurti, Ramesh, Williamsville, NY 14221 (US)
(74) Representative: VOSSIUS & PARTNER

(57) **Abstract**

Disclosed is a method of making a mixture of 1,4-dichlorobenzene and 1,2,4-trichlorobenzene in which 1,2,4-trichlorobenzene is the major component and 1,4-dichlorobenzene is the second major component. Benzene is reacted with about 2.4 to about 2.9 moles of chlorine gas per mole of benzene in the presence of a chlorination catalyst and an elemental sulfur cocatalyst, at a temperature of about 40 to about 90°C.

## Description

This invention relates to a method of making a mixture of chlorinated benzenes in which 1,2,4-trichlorobenzene is the major component and 1,4-dichlorobenzene is the second major component. In particular, it relates to the reaction of benzene with chlorine gas in the presence of a catalyst and an elemental sulfur cocatalyst.

The compound 1,4-dichlorobenzene (1,4-DCB) is used for the production of polyphenylenesulfides, and the compound 1,2,4-trichlorobenzene (1,2,4-TCB) is used to make herbicides and termite insecticides. Of those two chlorinated benzenes, 1,2,4-TCB is the most valuable commercially.

Traditionally, these compounds are prepared by chlorinating benzene in a continuous process, but that process has not been satisfactory for various reasons, including low yield, production of large amounts of undesirable byproducts, in particular, 1,2-dichlorobenzene (1,2-DCB) and 1,2,3-trichlorobenzene (1,2,3-TCB), and the need to recycle a large stream of monochlorobenzene. This recycling causes a low throughput of the desired 1,4-DCB and 1,2,4-TCB. Also, the separation of the unwanted byproducts requires a tedious distillation with low throughput.

It has been found a process for making a mixture of 1,4-DCB and 1,2,4-TCB, where 1,2,4-TCB is the major component and 1,2-DCB is the second major component, in a high combined yield with the production of relatively little 1,2-DCB and 1,2,3-TCB. It is possible to obtain a high ratio of 1,4-DCB to 1,2-DCB and of 1,2,4-TCB to 1,2,3-TCB by chlorinating benzene in the presence of a catalyst and elemental sulfur cocatalyst.

It has further been found that the proportions of 1,2-DCB and 1,2,4-TCB increase significantly at higher ratios of catalyst to cocatalyst. And, very unexpectedly, it has been found that higher proportions of 1,4-DCB and 1,2,4-TCB are produced when benzene is chlorinated instead of when chlorobenzene is chlorinated, even though chlorobenzene is the first intermediate formed when benzene is chlorinated.

In the process of this invention benzene is chlorinated with about 2.4 to about 2.9 moles of chlorine gas per mole of benzene, as measured by gas chromatography (GC). Extra chlorine gas should be used to make up for any chlorine loss that occurs. If less than 2.4 moles of chlorine is used, lower ratios of 1,4-DCB to 1,2-DCB and of 1,2,4-TCB to 1,2,3-TCB are obtained, and if more than 2.9 moles of chlorine are used, the yield of 1,4-DCB and of 1,2,4-TCB decreases and more tetrachlorobenzenes (tetra-CB) are formed; the preferred amount of chlorine is about 2.5 to about 2.85 moles per mole of benzene. The chlorination should be performed at a temperature of about 40 to about 90°C, although the reaction will occur at higher or lower temperatures.

The reaction must be performed in the presence of a chlorination catalyst. Suitable chlorination catalysts include ferric chloride (FeCl₃), antimony pentachloride (SbCl₅), aluminum trichloride (AlCl₃), cupric chloride (CuCl₂), zinc chloride (ZnCl₂), cobalt chloride (CoCl₂), nickel chloride (NiCl₂), molybdenum pentachloride (MoCl₅), and niobium pentachloride (NbCl₅). The preferred catalyst is ferric chloride because it is very effective and economical. About 0.1 to about 1 m mole of catalyst per mole of benzene should be used as less is less effective and more is usually unnecessary. The preferred amount of catalyst is about 0.3 to about 1 m mole per mole of benzene. While the catalyst can be formed in situ by, for example, reaction of the chlorine with an iron vessel to form ferric trichloride, this is not recommended because the reaction is difficult to control under these circumstances and may be hazardous.

An elemental sulfur cocatalyst is also required for this reaction. Other catalysts, such as sulfur compounds, do not produce the high yield of 1,4-DCB and 1,2,4-TCB, and high ratios of 1,4-DCB to 1,2-DCB and of 1,2,4-TCB to 1,2,3-TCB, that elemental sulfur does. The amount of elemental sulfur cocatalyst should be about 0.05 to about 1 m mole per mole of benzene, as less is less effective and more is usually unnecessary. The preferred amount of elemental sulfur cocatalyst is about 0.1 to about 1 m mole per mole of benzene. Significantly better 1,2,4-TCB/1,2,3-TCB and 1,4-DCB/1,2-DCB ratios are obtained when the mole ratio of FeCl₃/S is about 1, as is demonstrated in the examples that follow. Particularly preferred conditions for a ferric chloride catalyzed process are a reaction temperature of 40 to 90°C, a chlorine gas to benzene molar ratio of about 2.6, a ferric chloride catalyst to benzene ratio of about 1 m mole, and an elemental sulfur cocatalyst to benzene ratio of about 0.5 m mole.

The reaction proceeds about as rapidly as the chlorine is added and can be completed in about 6 hours. The course of the reaction can be followed by GC analysis of aliquots of the reaction mixture. In order to avoid or minimize chlorine breakthrough (i.e., unreacted chlorine), it is necessary to raise the temperature in stages during the process, particularly when the GC analysis shows no or very little monochlorobenzene at the reaction temperature. The product of the reaction is a mixture of chlorobenzenes, including 1,4-DCB, 1,2-DCB, 1,2,4-TCB, 1,2,3-TCB, and tetra chlorinated benzenes. Of the chlorinated benzenes present in the mixture, the major component (i.e., the component present at the highest weight percent) is 1,2,4-TCB and the second major component (i.e., the component present at the second highest weight percent) is 1,4-DCB. The weight ratio of 1,4-DCB to 1,2-DCB is typically greater than 50 and the weight ratio of 1,2,4-TCB to 1,2,3-TCB is typically greater than 20. The desired products, 1,4-DCB and 1,2,4-TCB, can be recovered from the product mixture by fractional distillation.

The following examples further illustrate this invention.

### Example 1

Chlorine gas was passed through benzene heated to an initial temperature of 60°C at various Cl₂/benzene molar ratios. After passing chlorine gas through the benzene for about 3.5 hours, the temperature of the reaction mixture was raised to 70°C and chlorine gas passage was continued. After about 1 hour, the temperature was raised to about 80°C and chlorine passing was continued for about 1 hour. A ferric chloride catalyst was used at benzene to ferric chloride molar ratios of 1600 to 2500 and various cocatalysts were used at benzene to cocatalyst molar ratios of 800 to 5000. The composition of the reaction mixture was analyzed by GC after various reaction times. Generally, the temperature of the reaction mixture was raised suitably to avoid or minimize Cl₂ breakthrough during the entire process. No benzene, monochlorobenzene, or 1,3-dichlorobenzene was found in any of the reaction mixtures. The following table gives the cocatalysts used and the results.

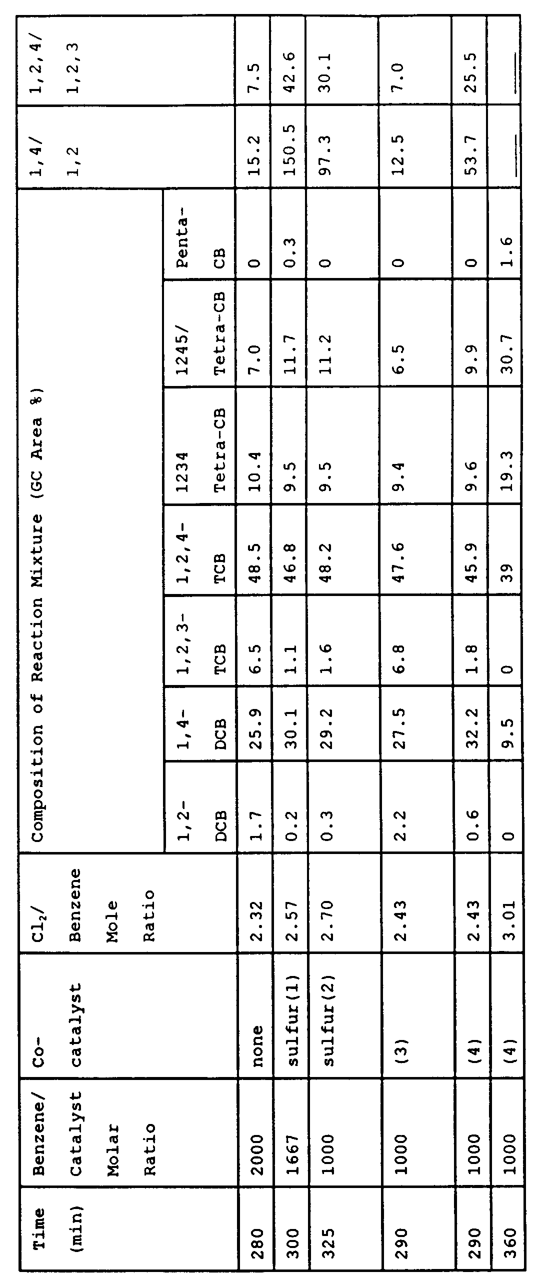

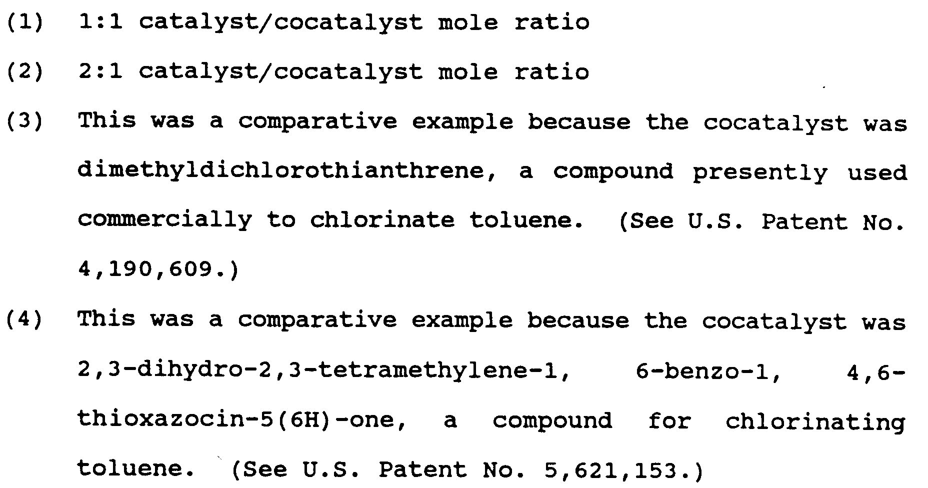

The table shows that when the cocatalyst was elemental sulfur, the yields of 1,4-DCB and 1,2,4-TCB were high and the ratio of 1,4-DCB to 1,2-DCB and of 1,2,4-TCB to 1,2,3-TCB was higher than when the cocatalyst was a sulfur compound or when no cocatalyst was used. The table also shows that significantly higher ratios of 1,4-DCB/1,2-DCB and 1,2,4-TCB/1,2,3-TCB were obtained at a catalyst to cocatalyst mole ratio of 1 than at a mole ratio of 2. At a chlorine to benzene mole ratio of 3.01 the yields of 1,4-DCB and 1,2,4-TCB decreased while the yield of tetrachlorobenzenes increased rapidly.

### Example 2 (Comparative)

Example 1 was repeated using chlorobenzene instead of benzene. The following table gives the results:

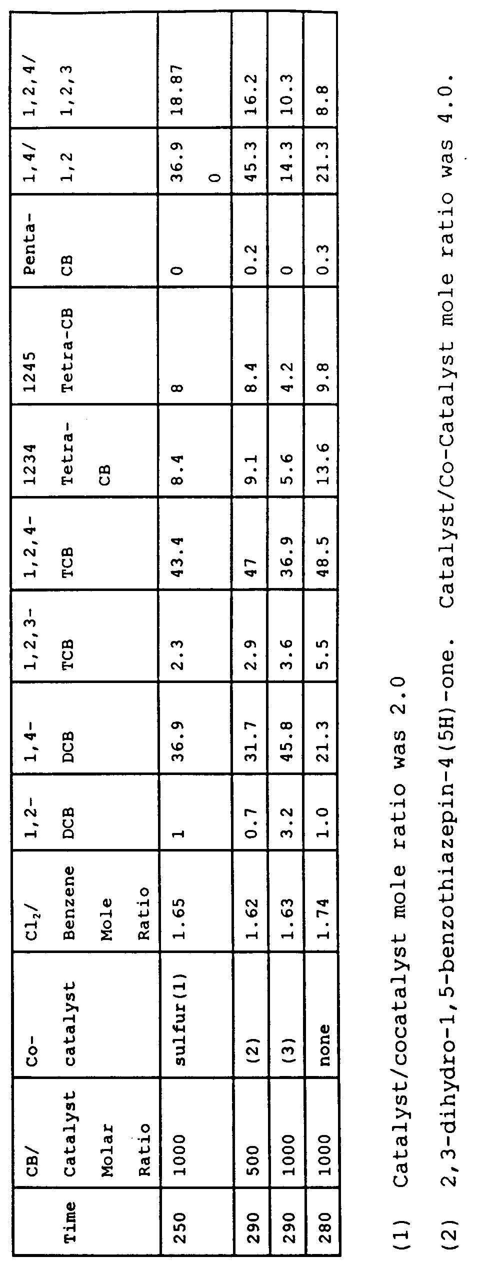

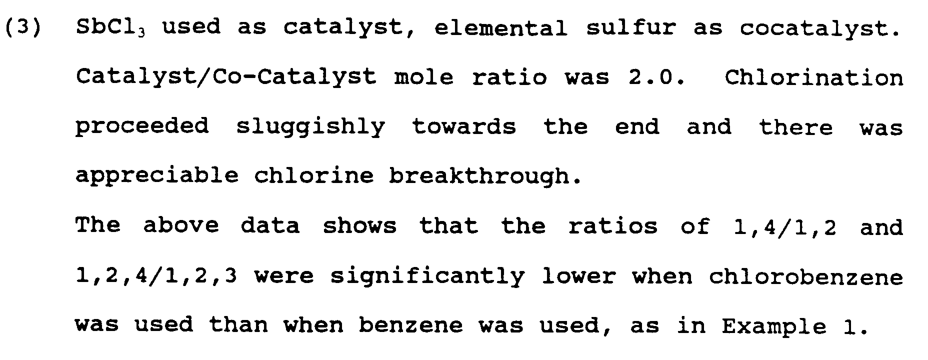

## Claims

1. A method of making a mixture of 1,4-dichlorobenzene and 1,2,4-trichlorobenzene in which 1,2,4-trichlorobenzene is the major component and 1,4-dichlorobenzene is the second major component comprising reacting benzene with about 2.4 to about 2.9 moles of chlorine gas per mole of benzene in the presence of an elemental sulfur cocatalyst and a chlorination catalyst selected from ferric chloride, antimony pentachloride, cupric chloride, zinc chloride, cobalt chloride, nickel chloride, molybdenum pentachloride, and niobium pentachloride.

2. A method according to Claim 1 performed at a temperature of about 40 to about 90°C.

3. A method according to Claim 1 or 2 wherein the amount of said chlorine gas is about 2.5 to about 2.85 moles per mole of said benzene.

4. A method according to any of Claims 1 to 3 wherein the amount of said chlorination catalyst is about 0.1 to about 1 m mole per mole of said benzene.

5. A method according to Claim 4 wherein the amount of said chlorination catalyst is about 0.3 to about 1 m mole per mole of said benzene.

6. A method according to any of Claims 1 to 5 wherein the amount of said elemental sulfur cocatalyst is about 0.05 to about 1 m mole per mole of said benzene.

7. A method according to Claim 6 wherein the amount of said elemental sulfur cocatalyst is about 0.1 to about 1 m mole per mole of said benzene.

8. A method according to any of Claims 1 to 7 wherein the mole ratio of chlorination catalyst to cocatalyst is greater than 1.

9. A method according to any of Claims 1 to 8 wherein said chlorination catalyst is ferric chloride.

10. A method according to any of Claims 1 to 8 wherein said chlorination catalyst is selected from antimony pentachloride, cupric chloride, zinc chloride, cobalt chloride, nickel chloride, molybdenum pentachloride, and niobium pentachloride.

11. A method of chlorinating benzene to produce 1,2,4-trichlorobenzene as the major product consisting essentially of reacting benzene with about 2.4 to about 2.9 moles of chlorine gas per mole of benzene in the presence of
(1) about 0.3 to about 1 m mole per mole of said benzene of a catalyst selected from ferric chloride, antimony chloride, antimony pentachloride, cupric chloride, zinc chloride, cobalt chloride, nickel chloride, molybdenum pentachloride, and niobium pentachloride; and
(2) about 0.1 to about 1 m mole elemental sulfur cocatalyst per mole of said benzene.

12. A method according to Claim 11 wherein the amount of said chlorine gas is about 2.5 to about 2.85 moles per mole of said benzene.

13. A method according to claim 11 or 12 wherein the mole ratio of said catalyst to said cocatalyst is greater than 1.

14. A method according to any of Claims 11 to 13 wherein said catalyst is ferric chloride.

15. A method according to any of Claims 11 to 14 performed at a temperature of about 40 to about 90°C.

16. A method according to any of Claims 11 to 13 wherein said catalyst is selected from antimony pentachloride, cupric chloride, zinc chloride, cobalt chloride, nickel chloride, molybdenum pentachloride, and niobium pentachloride.

17. A method of making a mixture of 1,4-dichlorobenzene, 1,2-dichlorobenzene, 1,2,4-trichlorobenzene, and 1,2,3-trichlorobenzene with a ratio of 1,4-dichlorobenzene to 1,2-dichlorobenzene greater than 50 and a ratio of 1,2,4-trichlorobenzene to 1,2,3-trichlorobenzene greater than 20, where said 1,2,4-trichlorobenzene is the major component and said 1,4-dichlorobenzene is the second major component, consisting of reacting benzene with about 2.5 to about 2.85 moles of chlorine gas per mole of benzene in the presence of about 0.3 to about 1 m mole per mole of said benzene of a ferric chloride catalyst and about 0.1 to about 1 m mole per mole of said benzene of an elemental sulfur cocatalyst, at a temperature of about 40 to about 90°C, where the mole ratio of ferric chloride catalyst to elemental sulfur cocatalyst is about 1.

18. A method according to Claim 17 wherein the amount of said ferric chloride catalyst is about 1 m mole per mole of said benzene.

19. A method according to Claim 17 or 18 wherein the amount of said elemental sulfur cocatalyst is about 0.5 m mole per mole of said benzene.

20. A method according to any of Claims 17 to 19 wherein the chlorine gas to benzene molar ratio is about 2.6.
